# EUROPEAN PATENT APPLICATION

(11) **EP 2 135 654 A1**
(43) Date of publication of application: **23.12.2009**
(21) Application number: 09007919.5
(22) Date of filing: 17.06.2009
(51) Int. Cl.: A63H 3/02

(54) **Stuffed toy filled with super absorbent polymer**

(30) Priority: 17.06.2008 KR 20080008084 U
(71) Applicant: Kim, Jin-Sung, Bundang-gu, Sungnam-si Gyeonggi-do, 463-811 (KR)
(72) Inventor: Kim, Jin-Sung, Bundang-gu, Sungnam-si Gyeonggi-do, 463-811 (KR)
(74) Representative: Albrecht, Thomas

(57) **Abstract**

Disclosed is a stuffed toy having a three-dimensional shape of various kinds of dolls or animals, which is fabricated by sewing a cloth (a fabric) and filling the inner space with a filler. The disclosed stuffed toy uses a bead-type high absorbent resin, instead of cotton or stuffings, as a filler, so as to vary the contraction and expansion state of the stuffed toy according to the amount of absorbed moisture of the high-absorbent resin without leakage of the high absorbent resin. The stuffed toy is fabricated by layering a water-permeable interior covering cloth 20 within a water-permeable exterior covering cloth 10 used for a conventional stuffed toy exterior covering prior to sewing in accordance with a toy shape, and filling a bead-type high absorbent resin 30 in an inner space S which is divided by the sewn exterior/interior covering cloths 10 and 20 in an amount corresponding to a volume of the inner space S, the bead-type high absorbent resin 30 expanding in a shape of a bead 31 by water absorption. Herein, the exterior/interior covering cloths 10 and 20 are water-permeable, at least the interior covering cloth 20 is a bead-impermeable cloth woven in such a manner that the bead 31 of the bead-type high absorbent resin 30 cannot permeate therethrough, and the sewing includes dense stitches so as to prevent the bead 31 from leaking.

## Description

### BACKGROUND OF THE INVENTION

### 1. Field of the invention

The present invention relates to a stuffed toy having a three-dimensional shape of various kinds of dolls or animals, which is fabricated by sewing a cloth (a fabric) and filling the inner space with a filler. More particularly, the present invention relates to a stuffed toy filled with a high absorbent resin, which uses a bead-type high absorbent resin, instead of cotton or stuffings, as a filler, so as to vary the contraction and expansion state of the stuffed toy according to the amount of absorbed moisture of the high-absorbent resin without leakage of the high absorbent resin.

### 2. Description of the Prior Art

As widely known in the art, a stuffed toy having a three-dimensional shape of various kinds of dolls or animals, which is fabricated by sewing the circumference of a cloth (a fabric) into a certain shape and filling the inner space with an appropriate filler, is made by the steps of cutting a cloth according to a predetermined pattern, backstitching the overlapped circumferences of the cloth while leaving a hole, turning over the backstitched cloth, filling the inner space with cotton, or the like, and sewing up the hole.

A stuffed toy is one of representative toys which have been traditionally popular because the stuffed toy has several advantages in that it provides a soft feeling of the cloth, is free from breakage or injury, and can be easily fabricated by using remnants and stuffings (cotton).

However, the stuffed toy has a disadvantage in that it is relatively inactive and monotonous and thus has a weak effect of stimulating curiosity, because its formed shape is maintained as it is. In order to complement such a disadvantage of the stuffed toy, a stuffed toy with a built-in electronic device (such as a sensor or a sound device) providing a sound or an active change has been widely commercially available.

Also, there is another limitation of the stuffed toy, in that playing with the toy in water is not suitable because its entire material (the filler as well as the outer cover) is made of fiber, such as cloth or cotton, and thus becomes damp by water-absorption and is difficult to dry.

### SUMMARY OF THE INVENTION

Accordingly, the present invention has been made to solve the above-mentioned problems occurring in the prior art. Unlike a conventional stuffed toy having a three-dimensional shape of various kinds of dolls or animals, which is fabricated by forming a shut space by the sewing of a cloth and filling the inner space with cotton or stuffings, the present invention provides a stuffed toy filled with a high absorbent resin, which uses a bead-type high absorbent resin, instead of cotton or stuffings, as a filler, so as to vary the contraction and expansion state of the stuffed toy according to the amount of absorbed (or evaporated) moisture of the high-absorbent resin without leakage of the high absorbent resin.

In accordance with an aspect of the present invention, there is provided a stuffed toy filled with a high absorbent resin.

The stuffed toy filled with the high absorbent resin according to the present invention is fabricated by layering a water-permeable interior covering cloth within a water-permeable exterior covering cloth used for a conventional stuffed toy exterior covering prior to sewing in accordance with a toy shape, and filling a bead-type high absorbent resin in an inner space (which is divided by the sewn exterior/interior covering cloths) in an amount corresponding to an inner space volume, the bead-type high absorbent resin expanding in a shape of a bead by water absorption.

Herein, the exterior covering cloth and the interior covering cloth are water-permeable, at least the interior covering cloth is a bead-impermeable cloth woven in such a manner that the bead of the bead-type high absorbent resin cannot permeate therethrough, and the sewing includes dense stitches so as to prevent the bead from leaking.

Preferably, the interior covering cloth includes a high-density cloth having a warp/weft yarns-thickness ranging from 20×20 to 60×60 yarn counts, and a warp/weft yarns-density ranging from 108×58 to 140×120 per inch.

Preferably, the sewing is carried out in a dense manner, for example, 7 to 20 stitches per inch.

Preferably, the bead-type high absorbent resin includes the bead having an average before-expansion diameter of 0.1 to 1mm, and is filled in an amount of about 1.2 to 10% with respect to the maximum weight of water to be input to the inner space.

Preferably, in the inner space, at least one additive of an aromatic agent or an anti-bacterial agent is input.

Preferably, the bead-type high absorbent resin is input to a capsule which is water-soluble or easily broken prior to filling.

### BRIEF DESCRIPTION OF THE DRAWINGS

The above and other objects, features and advantages of the present invention will be more apparent from the following detailed description taken in conjunction with the accompanying drawings, in which:
FIG. 1 is a photograph showing a perspective view of a stuffed toy according to an embodiment of the present invention;
FIG. 2 is a photograph showing a longitudinal cross sectional view of a stuffed toy according to an embodiment of the present invention, before and after the expansion of the stuffed toy;
FIG. 3 shows photographs illustrating the expansion/contraction steps of a stuffed toy according to an embodiment of the present invention;
FIG. 4 shows schematic longitudinal cross sectional views of a stuffed toy according to an embodiment of the present invention before and after the expansion of the stuffed toy; and
FIG. 5 shows photos of particles, in which FIG. 5A shows a bead-type high-absorbent resin suitable for a stuffed toy according to the present invention, and FIG. 5B shows a general high-absorbent resin.

### DETAILED DESCRIPTION OF THE EXEMPLARY EMBODIMENTS

Hereinafter, a high-absorbent-resin-filled stuffed toy according to the present invention will be described with reference to the accompanying drawings. The following embodiment is illustrative only, and the scope of the present invention is not limited thereto.

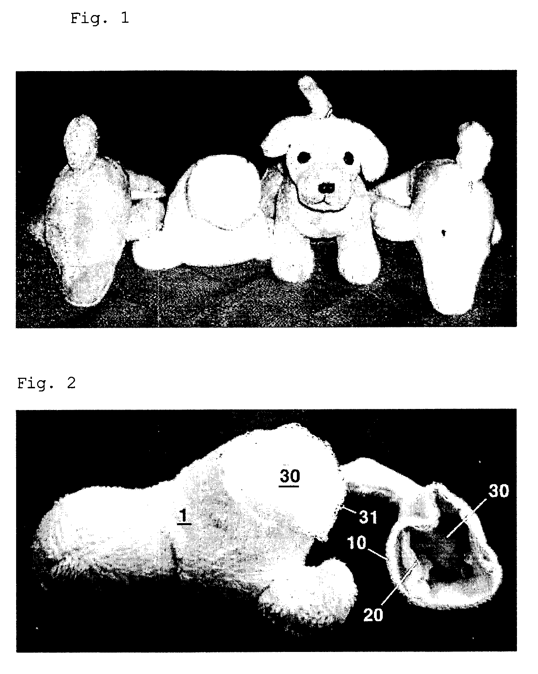

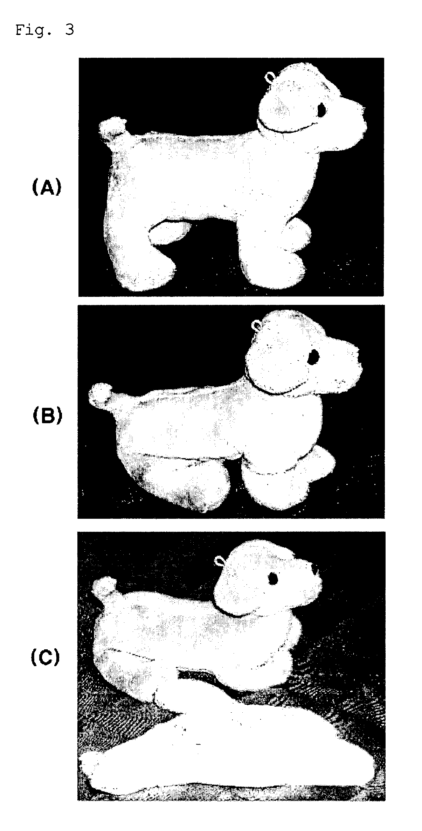

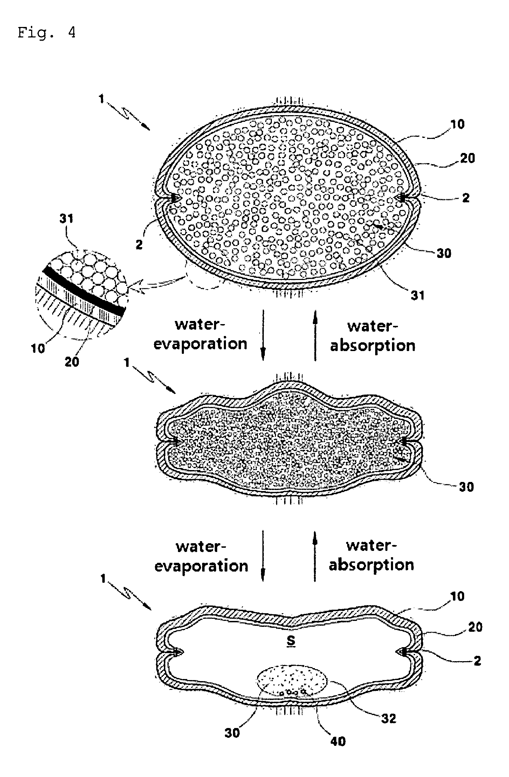

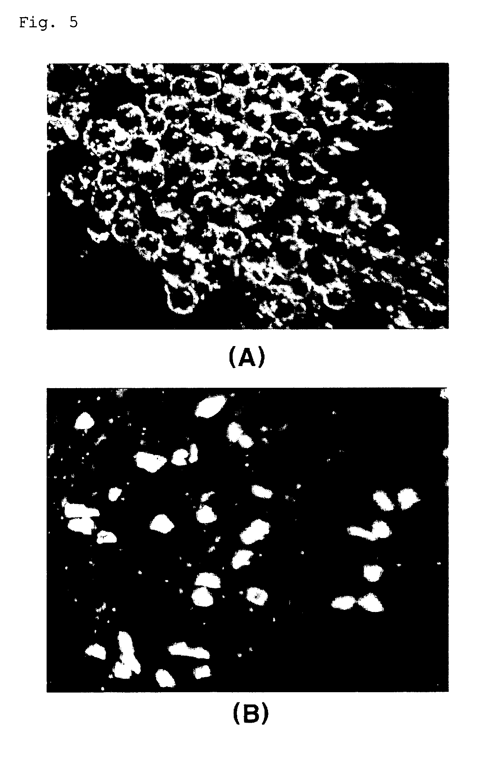

FIG. 1 is a photograph showing a perspective view of a stuffed toy according to an embodiment of the present invention, which includes dog/seal/dolphin stuffed toys. FIG. 2 is a photograph showing a longitudinal cross sectional view of a stuffed toy according to an embodiment of the present invention, in which the one on the right is a sectional view of the toy in a state where a high absorbent resin has not expanded (30), and the one on the left is a sectional view of the toy in a state where the high absorbent resin has expanded (31). FIG. 3 shows photographs illustrating the expansion/contraction steps of a stuffed toy according to an embodiment of the present invention, in which a toy in a completely expanded state (A) is gradually contracted by water evaporation with the passage of time, and especially the dolphin as shown in FIG. 3(C) is in a completely contracted state. FIG. 4 shows schematic longitudinal cross sectional views of a stuffed toy according to an embodiment of the present invention before and after the expansion of the stuffed toy. FIG. 5 shows photos of particles, in which FIG. 5A shows a bead-type high-absorbent resin suitable for a stuffed toy according to an embodiment of the present invention, and FIG. 5B shows a general high-absorbent resin.

As shown in FIGs. 1 to 4, a stuffed toy 1 filled with a high absorbent resin according to the present invention is a kind of stuffed toy having a shape of various kinds of dolls or animals, which is fabricated by forming a shut inner space by sewing of a cloth (a fabric) and filling the inner space with a filler, in the same manner as a conventional stuffed toy.

According to the characteristic of the present invention, a cloth includes a double cloth which includes an exterior covering cloth 10 and an interior covering cloth 20 selected in accordance with a certain condition, instead of a conventional single cloth.

As the exterior covering cloth 10, a conventional cloth which has been widely used for a stuffed toy and essentially has water-permeability may be used. For example, Velboa which is widely used as a cloth for a stuffed toy is preferable, and EF Velboa which is very pleasant to touch by using microfiber filament as yarn is more preferable.

As the interior covering cloth 20 layered on the inner surface of the exterior covering cloth 10, a high-density cloth which has water-permeability and is woven in such a manner that a bead 31 of the following bead-type high absorbent resin 30 cannot easily permeate therethrough, is used.

There is no particular limitation in the interior covering cloth 20 as long as the bead 31 of the bead-type high absorbent resin 30 cannot easily permeate through the cloth. For example, a high-density T/C (POLYESTER/RAYON) cloth which has been widely used as the lining of various kinds of clothes may be used.

More specifically, as the interior covering cloth 20, a high-density cloth (such as a T/C cloth) having a warp/weft yarns-thickness ranging from 20×20 to 60×60 spinning counts, and a warp/weft yarns-density ranging from 108×58 to 140×120 per inch may be used. Herein, when the yarn thickness is too great and out of the range, or the density is too high, water-permeation requires a long time, and also, when the density is less than the above mentioned range, leakage of the bead 31 may occur.

The double structure of a cloth used for the stuffed toy 1 according to the present invention, which includes the exterior covering cloth 10 having water-permeability and the interior covering cloth 20 having water-permeability and bead-impermeability, is to allow water to permeate into an inner space S via the exterior covering cloth 10 and the interior covering cloth 20 but to prevent the bead 31 of the bead-type high absorbent resin 30 from leaking via the fine high-density weaving of the interior covering cloth 20 while maintaining the stuffed toy's soft feeling by the exterior covering cloth 10.

Likewise, in sewing the exterior covering cloth 10 and the interior covering cloth 20 into the required shape, the sewing is required to be denser than that of a conventional stuffed toy, so as to prevent the bead-type high absorbent resin 30 from leaking via an opening between the stitches 2 of the sewing. For example, in the sewing to be applied in the present invention, in 1 inch, 7 to 20 stitches are preferable, and about 15 stitches are most preferable. By a high-performance sewing machine, it is possible to densely sew up to 20 stitches or more. However, a general sewing machine has limited performance and too many stitches may be non-effective in leakage prevention, and thus the sewing has only to prevent the leakage of the bead-type high absorbent resin 30.

In the inner space S of the stuffed toy 1 of the present invention, the bead-type high absorbent resin 30, such as sodium acrylate polymer, is filled.

As described in many documents, such as Korea Patent Registration No. 0492917 (June 1, 2005), "Process for preparing Bead-typed hydrogel", a high-absorbent resin is a kind of functional polymer which absorbs water in an amount of several tens times to several hundreds times its own weight and does not easily discharge the water by pressure to some extent, and is widely used for sanitary goods requiring moisture-absorption, such as a paper diaper, a sanitary napkin, a breast pad, etc.

In general, the high absorbent resin includes a synthetic material having a three-dimensional network structure by polymerization of a monomer having a hydrophilic group, such as -OH, -NH₂, -COOH, -SO₃H, with a cross-linking agent, and the hydrophilic monomer includes partially neutralized acrylic acid. In the synthesis, bulk polymerization, solution polymerization, inverse suspension polymerization, inverse emulsion polymerization, etc. may be used.

In preparing a high absorbent resin having a certain particle shape, inverse suspension polymerization or inverse emulsion polymerization is generally applied, and especially, the inverse suspension polymerization for polymerizing a water-soluble monomer through dispersion in a solvent is known as a preferred method for preparing a bead-type high absorbent resin.

A high absorbent resin has various particle shapes through expansion by moisture-absorption. Meanwhile, another high absorbent resin having amorphousness by expansion (see FIG. 5(B)) is not appropriate for the stuffed toy of the present invention because it may leak through the opening between stitches 2 of the interior covering cloth 20 and the exterior covering cloth 10 from the inner space S.

Therefore, in the stuffed toy 1 of the present invention, the bead-type high absorbent resin 30 which can have a bead-shape (a spherical shape, an oval shape, etc.) via expansion by moisture absorption is used (see FIG. 5 and drawings of Patent Registration No. 0492917).

FIG. 5 shows photos of particles of a bead-type high-absorbent resin and a general high-absorbent resin. Herein, the bead-type high-absorbent resin as shown in FIG. 5A is applicable to the present invention, while the amorphous (general) high-absorbent resin as shown in FIG. 5B is not preferable because it can cause leakage.

The bead-type high absorbent resin 30 applied to the present invention has a bead-shape by expansion via moisture absorption, and thus cannot permeate into the opening between the interior covering cloth 20 and the stitches 2. Even if it permeates through the opening, the amount is very small.

The resin 30 has a powdery shape (or a very-small-diameter bead shape) when it did not absorb water, and the resin 30 has a small-diameter bead shape when it partially absorbed water. However, against the expectation that the resin can leak by permeating through the opening between the interior covering cloth 20 and the stitches, the powder or small-diameter bead type high absorbent resin 30 does not leak from the inner space S, because it exists in a small volume with respect to the volume of the inner space S and thus does not give pressure to the interior covering cloth 20.

The diameter of the expanded bead 31 of the bead-type high absorbent resin 30 may be selected according to the density of the applied woven interior covering cloth 20. When the density of the interior covering cloth 20 is high, a relatively small diameter is allowable, and on the other hand, when the density is low, a relatively large diameter is required.

According to the test by the present invention designer, under the conditions of the above described interior covering cloth 20, the bead-type high absorbent resin 30 before expansion may have a large diameter to some extent. On the average, when the bead-type high absorbent resin 30 has a diameter ranging from 0.1 to 1mm, it is possible to avoid the leakage.

As described above, the bead-type high absorbent resin 30 is self-expanded by water-absorption several tens to hundreds times. In the case where too much of the bead-type high absorbent resin 30 is filled in the inner space S, when water is absorbed to the maximum, excessive expansion is caused, thereby highly increasing the pressure applied to the cloths 10 and 20. Thus, the possibility of leakage of the bead 31 or the rupture of the sewing is increased. On the other hand, in the case where a very little amount of resin 30 is filled, even when the expansion is maximized, the stuffed toy 1 may have a shape different from the original shape.

According to the test by the present invention designer, the appropriate filling amount of the bead-type high absorbent resin 30 was about 1.2 to 10% with respect to the weight of water absorbed in the inner space S although it depends on absorptance (expansivity) of the bead-type high absorbent resin 30. However, the filling amount of the bead-type high absorbent resin 30 may be appropriately adjusted according to the absorptance of the bead-type high absorbent resin 30 to be used, within a range where the maximum expansion extent of the stuffed toy 1 of the present invention is not too high.

The bead-type high absorbent resin 30 may be fabricated by the above described method, such as Patent Registration No. 0492917, or is commercially available. For example, from among several bead-type high absorbent resins 30 used by the present invention designer, a bead-type high absorbent resin of sodium acrylate polymer ((C₃H₄O₂)x. yNA) is an odorless white resin which has a bulk density of 0.9±0.1(g/ml), and is water-insoluble and harmless to the human body through intake or body contact. Especially, the bead-type high absorbent resin of sodium acrylate polymer can absorb water about 84 times and thus is preferable as a resin to be expanded so as to have a bead-shape.

In the use of the stuffed toy 1 according to the present invention, water is used, and thus a bacillus increase, etc. may cause an odor. Therefore, in order to prevent the odor, at least one additive 40 (an aromatic agent or anti-bacterial agent) in an appropriate amount (in general, a very small amount), together with the bead-type high absorbent resin 30, may be put in the inner space S. There is no particular limitation in such an additive 40, as long as it is conventionally widely used as an aromatic agent or anti-bacterial agent and is harmless to the human body. Of course, the odor may be easily removed through simple-washing by soap, etc.

The bead-type high absorbent resin 30 may be directly put in the inner space S which is divided by the cloths 10 and 20, or may be filled through an additionally prepared capsule 32. Herein, as the capsule 32, a water-soluble capsule or a capsule to be broken by pressure may be used.

In the application of the capsule 32, the capacity of the capsule 32 may be adjusted to a fixed amount of the bead-type high absorbent resin 30. Thus, according to a size of the stuffed toy 1 of the present invention, it is possible to easily adjust the amount of bead-type high absorbent resin 30 to be input into the toy to a fixed amount. Also, it is possible to prevent the bead-type high absorbent resin 30 from moving freely within the inner space S during the distribution of the stuffed toy 1 of the present invention. Once water is input to the stuffed toy 1 of the present invention, the capsule 32 is melted or broken.

The stuffed toy 1 as described above is in a powder state before water-absorption, and is in a paste (or gel) state after water-absorption. Accordingly, while a conventional high absorbent resin was not considered as the stuffed toy filler, the bead-type high absorbent resin 30 may be used as the filler for the stuffed toy according to the present invention by the application to the toy having a double structure including the exterior covering cloth 10 (which has water-permeability) and the interior covering cloth 20 (which has water-permeability/and bead-impermeability).

The stuffed toy 1 of the present invention shows a change in a contraction/expansion state and various postures, according to the amount of moisture absorbed/evaporated by/from the filled bead-type high absorbent resin. Thus, the stuffed toy has an advantage in that it provides interest in the expansion/contraction and can have various shapes. Also, it is possible to play with the stuffed toy in water while a conventional stuffed toy was not appropriate for water-play.

According to the test on the stuffed toy 1 by the present invention designer, as shown in FIG. 1, when the stuffed toy 1 in a complete contraction state (where moisture was almost not absorbed by the high absorbent resin 30) was immersed in water, it took about 1 to 3 minutes to reach a complete expansion state where the moisture was absorbed to the maximum. Meanwhile, it took several hours to one week or more, according to an evaporation condition, to reach the complete contraction state again by evaporating the water from the stuffed toy 1 in the complete expansion state.

In the stuffed toy 1 of the present invention, since water is applied to a fabric (the cloths 10 and 20), it is expected that a damp cloth may give the hand an unpleasant feeling. However, actually, since the high absorbent resin 30 absorbs water in the cloth to the maximum, there is no dampness which causes an unpleasant feeling. Also, during the process where the water absorbed in the high absorbent resin 30 is gradually evaporated, heat absorption occurs, thereby giving a cool feeling to the touch.

Also, after water-absorption and expansion of the stuffed toy 1 of the present invention, when the stuffed toy is frozen and then thawed at room temperature, the heat insulation of the exterior covering cloth 10 and the interior covering cloth 20 prevents dew condensation from occurring and allows the frozen water to gradually thaw for a long time. Accordingly, it is possible to keep the stuffed toy 1 in a cool state for a long time, in which the stuffed toy is not damp or too cold to touch. Therefore, the stuffed toy 1 of the present invention in a frozen state can be simply used for cooling heat, and also can be used as a cold-pack or to bring-down a fever.

When, besides the high absorbent resin 30, the additive 40 (an aromatic agent and/or anti-bacterial agent) in an appropriate amount is included as the filler of the stuffed toy 1 of the present invention, it is possible to minimize a bacillus increase and an odor caused by the use of water. Moreover, the stuffed toy when placed in a car or indoors may be used as an aromatic toy.

When the stuffed toy 1 of the present invention is provided to a user, it is preferable that the amount (a maximum or average amount) of moisture capable of being absorbed by the stuffed toy 1 is variously displayed on the package, so that the user can refer to the information.

The stuffed toy filled with the high absorbent resin 30 according to the present invention has the following effects.

First, since the stuffed toy according to the present invention uses a bead-type high absorbent resin, instead of cotton or stuffings, as a filler, the toy can be in a contraction/expansion state changeable according to the amount of moisture absorbed in the bead-type high absorbent resin, thereby showing various postures. Therefore, it is possible to provide interest by the expansion/contraction and variously changeable shapes.

Second, in the stuffed toy according to the present invention, a water-permeable and bead-impermeable interior covering cloth is layered within a conventional water-permeable cloth (which has been widely used for a stuffed toy) as an exterior covering cloth, and a selected bead-type high absorbent resin is applied as a filler, thereby allowing water to freely permeate through the exterior/interior covering cloths while preventing the bead-type high absorbent resin from leaking through the interior covering cloth. In other words, it is possible to apply a high-absorbent resin (which was not applicable) to a stuffed toy as a filler.

Third, although the stuffed toy according to the present invention includes a fabric (the exterior/interior covering cloths) and is used with water, there is, on the cloth surface, no dampness causing an unpleasant feeling due to water absorption by the bead-type high absorbent resin. Besides, during the process where the water absorbed in the bead-type high absorbent resin is gradually evaporated according to ambient temperature, heat absorption occurs, thereby giving a cool feeling to the touch. Therefore, it is possible to play with the stuffed toy in water, unlike a conventional stuffed toy which was unsuitable for water-play.

Fourth, in the stuffed toy according to the present invention, an aromatic agent and/or anti-bacterial agent in an appropriate amount, in addition to the bead-type high absorbent resin, may be added, thereby minimizing a bacillus increase and an odor caused by the use of water. Moreover, the stuffed toy when placed in a car or indoors may be used as an aromatic toy.

Fifth, when the stuffed toy of the present invention is frozen and then thawed at room temperature, the heat insulation of the exterior covering cloth and the interior covering cloth prevents dew condensation from occurring and allows the frozen water to gradually thaw for a long time. Thus, it is possible to keep the stuffed toy in a cool state for a long time, in which the stuffed toy is not damp or too cold to touch. Accordingly, the stuffed toy of the present invention in a frozen state can be simply used for cooling the heat, and also can be used as a cold-pack or to bring-down a fever.

Although an exemplary embodiment of the present invention has been described for illustrative purposes, those skilled in the art will appreciate that various modifications, additions and substitutions are possible, without departing from the scope and spirit of the invention as disclosed in the accompanying claims.

## Claims

1. A stuffed toy filled with a high absorbent resin, which is fabricated by layering a water-permeable interior covering cloth 20 within a water-permeable exterior covering cloth 10 used for a conventional stuffed toy exterior covering prior to sewing in accordance with a toy shape, and filling a bead-type high absorbent resin 30 in an inner space S which is divided by the sewn exterior covering cloth 10 and interior covering cloth 20 in an amount corresponding to a volume of the inner space S, the bead-type high absorbent resin 30 expanding in a shape of a bead 31 by water absorption,
wherein the exterior covering cloth 10 and the interior covering cloth 20 are water-permeable, at least the interior covering cloth 20 is a bead-impermeable cloth woven in such a manner that the bead-type high absorbent resin 30 having a before-expansion diameter of 0.1 to 1mm cannot permeate therethrough, sewing of the interior covering cloth 20 comprises 7 to 20 stitches per inch so as to prevent the bead-type high absorbent resin 30 from leaking, and the bead-type high absorbent resin 30 is filled in an amount of about 1.2 to 10% with respect to a maximum weight of water to be input to the inner space S.

2. The stuffed toy as claimed in claim 1, wherein in the inner space S, at least one additive 40 of an aromatic agent or an anti-bacterial agent is input.

3. The stuffed toy as claimed in claim 1, wherein the bead-type high absorbent resin 30 is input to a capsule 32 which is water-soluble or easily broken prior to filling.
